Europäisches Patentamt

European Patent Office

Office européen des brevets

(11) Veröffentlichungsnummer: **0 362 484**

**A2**

(12) **EUROPÄISCHE PATENTANMELDUNG**

(21) Anmeldenummer: **89112195.6**

(22) Anmeldetag: **04.07.89**

(51) Int. Cl.⁵: **A61M 5/24 , A61M 5/14**

(30) Priorität: **05.10.88 DE 3833821**

(43) Veröffentlichungstag der Anmeldung:
**11.04.90 Patentblatt 90/15**

(84) Benannte Vertragsstaaten:
**AT BE CH DE ES FR IT LI LU NL**

(71) Anmelder: **B. Braun Melsungen AG**
**Carl-Braun Strasse**
**D-3508 Melsungen(DE)**

(72) Erfinder: **Austenat, Elke, Dr.**
**Dudenstrasse 6**
**D-1000 Berlin 61(DE)**
Erfinder: **Boxberger, Michael, Dr.**
**Zur Schlade 47**
**D-3508 Melsungen(DE)**

(74) Vertreter: **Selting, Günther, Dipl.-Ing. et al**
**Patentanwälte von Kreisler, Selting, Werner**
**Deichmannhaus am Hauptbahnhof**
**D-5000 Köln 1(DE)**

(54) **Injektionsvorrichtung.**

(57) Die Injektionsvorrichtung, die insbesondere für die Verabreichung von Insulin an Diabetiker geeignet ist, enthält in einem Gehäuse (10) ein von einem Mikroprozessor (MP) gesteuertes Dosiergerät, dessen Pumpe (18) das Medikament aus einer Ampulle (13) ausdrückt. Die Programmierung des Injektionszeiten und der Medikamentengaben wird vom Arzt durchgeführt. Beim Ertönen eines akustischen Signaltones setzt der Patient die Kanüle (29) auf, führt sie zur Injektionsstelle und die Injektion erfolgt durch manuelle Betätigung eines Betätigungselementes (15). Die laufende Injektion wird an einem Display (11) angezeigt und ihre Beendigung wird durch einen Signalton kenntlich gemacht. Der Mikroprozessor (MP) ermöglicht eine genaue Einhaltung der Injektionszeiten und der Bolusmengen.

FIG.2

## Injektionsvorrichtung

Die Erfindung betrifft eine Injektionsvorrichtung der im Oberbegriff des Patentanspruchs 1 angegebenen Art.

Diabetiker verwenden für die Insulinzufuhr Einmalspritzen oder Injektionsvorrichtungen mit gesteuerter Medikamentenzufuhr. Bei den Injektionsvorrichtungen unterscheidet man zwei Typen: Die Infusionspumpe, bei der der Patient das Dosiergerät in seiner Kleidung mitführt und das Dosiergerät eine ständig arbeitende Pumpe (kontinuierliche Insulinapplikation) enthält, die Insulin durch einen Schlauch zu einer Kanüle fördert, welche in den Körper des Patienten eingeführt ist, und einen sogenannten Pen (diskontinuierliche Insulinapplikation). Die Insulinpumpe kommt aufgrund der erforderlichen Stoffwechselkontrollen nur für einen sehr kleinen Teil der Diabetiker in Betracht. Das ständige Tragen einer Pumpe ist lästig und wird von vielen Patienten abgelehnt. Der Pen ist eine Injektions vorrichtung, die etwa die Form und Größe eines Kugelschreibers hat und bei durch manuellen Druck auf einen Knopf oder Drehelement ein Teil des Inhalts, der im Pen-Gehäuse enthaltenen Ampulle, durch die am vorderen Gehäuseende befestigte Kanüle ausgedrückt wird. Der Patient führt die Punktion mit der Kanüle des Pens durch und betätigt anschließend den Druckknopf für die Injektion. Dabei gelangt eine voreinstellbare Insulinmenge in den Körper. Bei vielen Patienten stellt die exakte Dosiseinstellung der Bolusmenge ein großes Problem dar.

Der Erfindung liegt die Aufgabe zugrunde, eine Injektionsvorrichtung der im Oberbegriff des Patentanspruchs 1 angegebenen Art zu schaffen, die pünktliche Injektionen genau vorgegebener Bolusmengen auf einfache Weise und ohne die Gefahr menschlicher Fehler ermöglicht, jedoch nicht das dauernde Tragen eines Infusionssystems am Körper erfordert.

Die Lösung dieser Aufgabe erfolgt erfindungsgemäß mit den im kennzeichnenden Teil des Patentanspruchs 1 angegebenen Merkmalen.

Die erfindungsgemäße Injektionsvorrichtung vereinigt die Vorteile computergesteuerter Insulinpumpen mit denjenigen von Pen-Injektionsvorrichtungen. Die Injektion einschließlich der Punktion und des Auslösens der Medikamentengabe wird vom Patienten selbst manuell durchgeführt, wodurch das ständige Tragen eines Infusionssystems am Körper entfällt. Andererseits werden die Zeitpunkte der Bolusgabe und die Bolusmengen von dem Mikroprozessor überwacht und gesteuert. Zu den vorgegebenen Zeitpunkten erzeugt der Mikroprozessor akustische Alarmsignale. Der Patient setzt dann die Nadel auf, führt sie zur Injektionsstelle und die Injektion erfolgt durch Betätigung des Betätigungsorgans, das vorzugsweise als Druckknopf ausgebildet ist. Der zeitliche Verlauf der Injektion und die Bolusmenge werden von dem Mikroprozessor gesteuert. Auf einem am Gehäuse vorgesehenen Display kann die Tatsache angezeigt werden, daß die Injektion gerade erfolgt und noch nicht abgeschlossen ist. Nach erfolgter Injektion zieht der Patient die Nadel wieder heraus. Die Nadel kann anschließend mit einer Schutzkappe bedeckt oder in das Gehäuse eingezogen werden und das Gerät kann bis zur nächsten Injektion abgelegt oder in der Kleidung untergebracht werden.

Bei der erfindungsgemäßen Injektionsvorrichtung erfolgt keine Dauerinfusion, sondern die Injektionen werden in zeitlichen Abständen vom Patienten selbst durchgeführt. Der Injektionsvorgang selbst wird durch den Mikroprozessor überwacht und gesteuert.

Gemäß Anspruch 2 ist vorgesehen, daß nach erfolgter Injektion ein akustisches Ende-Signal erzeugt wird. Der Patient erhält dadurch eine klare Angabe darüber, wann er die Nadel herausziehen kann.

Vorzugsweise ist die Injektionsvorrichtung gemäß Anspruch 3 durch ein Programmiergerät programmierbar. Über dieses Programmiergerät verfügt der Arzt, der die Injektionszeiten und die zugehörigen Bolusmengen eingibt. Der Mikroprozessor enthält eine Zeituhr, um die Injektionszeiten zu erkennen und das Alarmsignal erzeugen zu können. Auf dem Display können weiterhin Hinweise über die noch verfügbare Batterieleistung und die Menge des in der Ampulle noch vorhandenen Medikamentenvorrats angegeben werden.

Mit den Merkmalen des Anspruchs 4 kann das Gerät für unterschiedliche Ampullentypen eingesetzt werden. Für jeden Ampullentyp ist ein spezieller Wechseleinsatz vorgesehen, der den Aufnahmeraum für die Ampulle abschließt. Dieser Wechseleinsatz trägt eine Kodierung, die von einem gehäuseseitigen Sensor erkannt wird. Dem Mikroprozessor wird eine Information über den Typ des Wechseleinsatzes und somit über den zugehörigen Ampullentyp mitgeteilt. Der Mikroprozessor kann daraufhin die Vorschubintervalle, mit denen der Kolben der Ampulle bei den einzelnen Bolusgaben vorgeschoben wird, entsprechend dem Ampullentyp variieren und somit bei jeder Bolusgabe die voreingestellte Bolusmenge ausdrücken.

Eine weitere Möglichkeit besteht darin, an dem Gehäuse eine Eingabevorrichtung vorzusehen, über die der Patient oder der Arzt die benötigte Medikamentendosierung einstellen können. Die eingestellten Daten können auf Tastendruck abgerufen werden.

Die erfindungsgemäße Injektionsvorrichtung eignet sich insbesondere für die Insulindosierung bei Diabetikern, sie ist jedoch auch zur Applikation anderer Medikamente geeignet, wie Heparin, Papaverin oder zur patientenkontrollierten Schmerztherapie. Falls der Patient die Dosis am Gerät selbst einstellen kann, sollte vom Arzt eine Dosisbegrenzung einprogrammiert werden können.

Im folgenden wird unter Bezugnahme auf die Zeichnungen ein Ausführungsbeispiel der Erfindung näher erläutert.

Es zeigen:

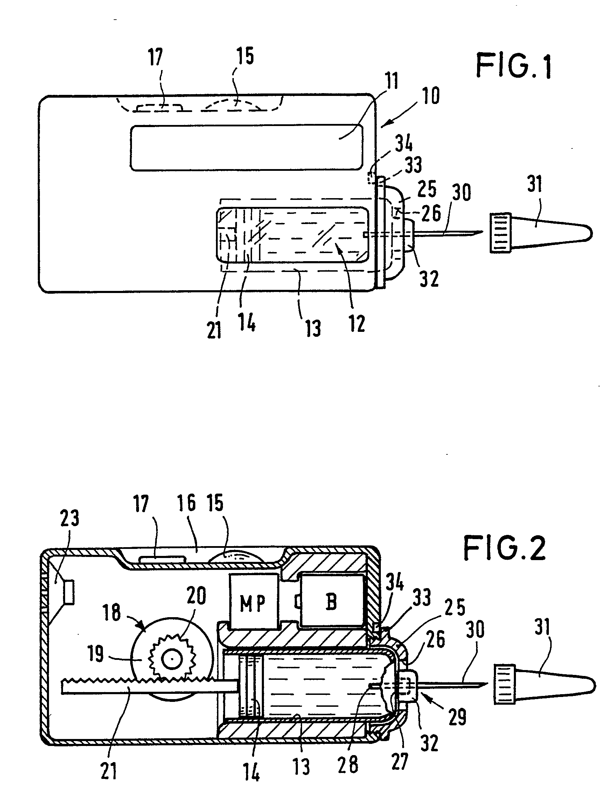

Fig. 1 eine Ansicht der Injektionsvorrichtung und

Fig. 2 eine Ansicht des geöffneten Gehäuses der Injektionsvorrichtung.

Die Injektionsvorrichtung weist ein flaches Gehäuse 10 auf, das etwa die Flächenabmessungen einer Steckkarte hat und bequem in einer Jackentasche mitgeführt werden kann. Außen am Gehäuse sind ein LCD-Display 11 und ein Sichtfenster 12 vorgesehen. Durch das Sichtfenster 12 ist die Ampulle 13 sichtbar, die das Medikament enthält und deren Raum durch den Kolben 14 abgeschlossen ist, der ebenfalls sichtbar ist. Anhand der Stellung des Kolbens 14 kann die Menge des noch in der Ampulle 13 befindlichen Medikaments erkannt werden.

Am Gehäuse 10 ist ferner das Betätigungselement 15 in Form eines Druckknopfes angebracht. Dieses Betätigungselement 15 ist in einer Mulde 16 des Gehäuses untergebracht, so daß es nicht versehentlich betätigt werden kann. Neben dem Betätigungselement 15 ist ein Steckverbinder 17 für den Anschluß eines externen Programmiergerätes angeordnet.

Im Inneren des Gehäuses 10 befindet sich die Pumpe 18, die aus dem Elektromotor 19, dem vom Elektromotor angetriebenen Ritzel 20 und der vom Ritzel 20 angetriebenen Zahnstange 21 besteht. Das vordere Ende der Zahnstange 21 drückt gegen die Rückseite des Kolbens 14 und schiebt diesen Kolben in der Ampulle 13 zum Ausdrücken des Medikaments vor.

Ferner sind im Gehäuse 10 der Mikroprozessor MP und die Batterie B untergebracht. Außerdem enthält das Gehäuse einen akustischen Signalerzeuger 23.

Der Aufnahmeraum 24 für die auswechselbare Ampulle 13 ist an einer Stirnseite des Gehäuses 10 mit einem angeschraubten Wechseleinsatz 25 verschlossen. Dieser Wechseleinsatz hat die Form einer Kappe, welche auf den Typ der betreffenden Ampulle 13 abgestimmt ist. Der Wechseleinsatz 25 enthält eine Öffnung 26, durch die der Hals der Ampulle hindurchragt. Dieser Ampullenhals ist mit einem Septum 27 verschlossen, das von der rückwärtigen Nadel 28 der Doppelkanüle 29 durchstochen werden kann. Die vordere Nadel 30 der Doppelkanüle wird in den Körper des Patienten eingestochen. Diese Nadel 30 ist mit einer Nadelschutzkappe 31 bedeckt, welche auf das die Doppelkanüle 29 enthaltende Anschlußstück 32 aufgesteckt wird.

Der Wechseleinsatz 25 ist mit einer charakteristischen Markierung 33 versehen, die von einem am Gehäuse 10 vorgesehenen Sensor 34 erkannt werden kann. Der Sensor 34 teilt den Typ des am Gehäuse befestigten Wechseleinsatzes dem Mikroprozessor MP mit und dieser berechnet unter Berücksichtigung der Eigenschaften der zugehörigen Ampulle 13 denjenigen Weg, den der Kolben 14 zur Injektion der vorgesehenen Bolusmenge zurücklegen muß.

Die Programmierung der Injektionszeiten und der Bolusmengen wird vom Arzt durchgeführt. Zu den Injektionszeiten bewirkt der Mikroprozessor MP die Erzeugung eines Alarmsignals durch den Signalgeber 23. Der Patient setzt die Doppelkanüle 29 auf die Ampulle auf, wobei die Nadel 28 das Septum 27 durchstößt. Nach Entfernen der Nadelschutzkappe 31 wird die Nadel zur Injektionsstelle geführt. Die Injektion erfolgt durch Betätigung des Betätigungselementes 15, das von dem Mikroprozessor vorbereitet wurde. Bevor das Alarmsignal erzeugt wurde, bleibt eine Betätigung des Betätigungselementes 15 wirkungslos. Erst nach Erzeugung des Alarmsignals wird bei Betätigung des Betätigungselementes 15 die Pumpe 18 in Betrieb gesetzt und die Erzeugung des Alarmsignals wird beendet. An dem Display 11 erscheint eine Anzeige, die angibt, daß die Injektion läuft. Nachdem die Injektion beendet ist, erfolgt am Display 11 eine andere Anzeige, die darauf hinweist, daß die Injektion beendet ist und ferner wird ein akustisches Ende-Signal erzeugt.

Bei einer anderen Ausführungsform der Injektionsvorrichtung ist die Dosiervorrichtung so ausgebildet, daß sie auch für den Typ I-Diabetiker eine intensivierte Insulintherapie ermöglicht. Dazu ist eine Eingabevorrichtung vorgesehen, an der der Diabetiker die benötigte Insulindosierung einstellen und auf einfachen Tastendruck abrufen kann, eine akustische Bestätigung der Eingabe und visuelle Überprüfung über das Display wird ermöglicht.

Die Injektionsvorrichtung zeichnet sich insbesondere durch eine einfache Bedienbarkeit aus, bei der Fehler praktisch nicht vorkommen können.

**Ansprüche**

1. Injektionsvorrichtung mit einer in einem Gehäuse (10) untergebrachten Dosiervorrichtung, welche unter Steuerung durch einen Mikroprozessor (MP) mit einer elektrisch betriebenen Pumpe (18) aus einer Ampulle (13) kontrollierte Mengen eines Medikamentes ausstößt, und mit einer Kanüle (29) zum Injizieren des Medikaments in den Körper, **dadurch gekennzeichnet,** daß die Dosiervorrichtung derart ausgebildet ist, daß die Pumpe (18) auf manuelle Betätigung eines Betätigungselementes (15) eine voreingestellte Bolusmenge ausstoßen kann, und daß der Mikroprozessor (MP) zu vorbestimmten Zeiten Alarmsignale erzeugt und daraufhin das Betätigungselement (15) zum Ausstoßen der voreingestellten Bolusmenge vorbereitet.

2. Injektionsvorrichtung nach Anspruch 1, dadurch gekennzeichnet, daß der Mikroprozessor (MP) nach erfolgter Injektion ein optisches und/oder akustisches Ende-Signal erzeugt.

3. Injektionsvorrichtung nach Anspruch 1 oder 2, dadurch gekennzeichnet, daß am Gehäuse (10) ein Steckverbinder (17) für den Anschluß eines Programmiergerätes vorgesehen ist, über das dem Mikroprozessor (MP) die Alarmzeiten und Bolusmengen eingegeben werden können.

4. Injektionsvorrichtung nach einem der Ansprüche 1 bis 3, dadurch gekennzeichnet, daß an dem Gehäuse (10) verschiedene Wechseleinsätze (25) zu be festigen sind, die den Aufnahmeraum (24) für die Ampullen (13) begrenzen und auf unterschiedliche Ampullentypen abgestimmt sind, und daß ein am Gehäuse (10) vorgesehener Sensor (34) den Typ des Wechseleinsatzes (25) erkennt und eine entsprechende Information an den Mikroprozessor (MP) liefert.

5. Injektionsvorrichtung nach einem der Ansprüche 1 bis 4, dadurch gekennzeichnet, daß die Kanüle (29) direkt und ohne Schlauch mit der Ampulle (13) verbindbar ist.

FIG.1

FIG.2